# EUROPEAN PATENT APPLICATION

(11) **EP 3 381 828 A1**
(43) Date of publication of application: **03.10.2018**
(21) Application number: 17170876.1
(22) Date of filing: 12.05.2017
(51) Int. Cl.: B65D 25/10, B01L 9/06, A61M 5/00

(54) **SUPPORTING STRUCTURE FOR SUPPORTING A PLURALITY OF CONTAINERS FOR SUBSTANCES FOR MEDICAL, PHARMACEUTICAL OR COSMETIC APPLICATIONS, COMBINED SUPPORTING STRUCTURE AND TRANSPORT AND PACKAGING CONTAINER COMPRISING THE SAME**

(30) Priority: 31.03.2017 IN 201721011580
(71) Applicant: Schott Kaisha Pvt. Ltd., Fort Mumbai, Maharashtra 400023 (IN)
(72) Inventor: Narvekar, Anil Narayan, 403707 Goa (IN); Potdar, Pratul Prakash, 396210 Nani Dama (IN)
(74) Representative: 2K Patentanwälte Blasberg Kewitz & Reichel

(57) **Abstract**

The invention discloses a supporting structure for supporting a plurality of medication containers (5), comprising an upper side (10) and a bottom side opposite to the upper side, a plurality of cup-shaped receptacles (13) for accommodating the containers, and a plurality of resilient positioning devices (18) for positioning and centering the containers in the receptacles, wherein the receptacles (13) extend in a direction perpendicular to the upper side (10), and supporting protrusions (22) are provided at the bottom ends of the receptacles for retaining the containers in their axial direction when accommodated in the receptacles.

According to the invention, the positioning devices are formed as resilient webs (18) protruding from inner side walls of the receptacles (13) radially inwards into the receptacles, each of the receptacles comprising at least two resilient webs (18) disposed at equiangular distances to each other and distributed along the inner side wall (15) of the receptacles, for centering the containers in the receptacles.

The supporting structure is configured for supporting a variety of medication containers of different outer diameters. Two or more supporting structures can be stacked on above the other if no medication containers are accommodated, which helps to save space during processing.

## Description

### Field of Invention

The present invention generally relates to a nested packaging solution for medication containers and relates in particular to a supporting structure for supporting a plurality of containers for substances for medical, pharmaceutical or cosmetic applications, particularly of vials.

### Background of Invention

For the processing of medication containers, such as vials or cartridges, so-called nested packaging solutions become more and more popular, where for transport and processing purposes a plurality of medication containers are concurrently supported by a supporting structure (so-called nest), which is accommodated in a transport and packaging container which is sterile sealed. The supporting structure precisely defines a regular arrangement of the containers, for example a matrix arrangement along rows and along columns extending perpendicular thereto. For the further processing of the containers, e.g. for filling of medication containers with a drug or solution, the supporting structures simply need to be removed from the transport and packaging container. As the containers are supported by the supporting structure at precisely defined positions, this has advantages in the automated further processing of the containers since the containers can be transferred to processing stations at controlled positions and in a predetermined arrangement, for example to processing machines, robots or the like. The downstream processing station will then know at what position and in what arrangement the containers to be processed are arranged.

Such a nested packaging solution is disclosed e.g. in WO 2009 015862 A1, where resilient holding tabs press firmly against the constricted neck portions at the upper ends of the vials to retain the vials by friction. The outer diameter of the vials is used basically as an auxiliary contour for fixing the vials on the supporting structure. Therefore, the use of such supporting structures is not flexible enough for vials having significantly different outer diameters.

A similar supporting structure is disclosed in WO 2011 135085 A1, where the medication containers are accommodated in receptacles having retaining protrusions protruding radially inwards into the receptacles at their bottom ends for retaining the medication containers in axial direction. In this supporting structure the dimensions of the receptacles need to be matched precisely to the outer diameter of the medication containers to be accommodated. A rattle-free supporting of medication containers of significantly different outer diameters is not possible with one and the same supporting structure.

The medication containers are usually further processed in a clean room offering only limited space. Often, for the further processing the vials are removed from the supporting structures temporarily or finally. This requires the space-saving storage of a plurality of supporting structures. Also, the disposal or re-use of such supporting structures generally favors space-saving solutions.

### Summary of Invention

It is an object of the present invention to further enhance a supporting structure for concurrently supporting a plurality of containers for medical, pharmaceutical or cosmetic applications, particularly of glass or plastic vials, so that the containers can be retained in a simple and reliable manner. According to a preferred further aspect of the present invention such a supporting structure shall be configured in particular for a space-saving storage of a plurality of supporting structures.

According to a further aspect of the present invention there are to be provided an enhanced combined supporting structure accommodating a plurality of containers, an enhanced assembly of such supporting structures and a transport and packaging container accommodating such an enhanced supporting structure.

These problems are solved by a supporting structure as claimed by claim 1, by a combined supporting structure as claimed by claim 11, by an assembly of such supporting structures as claimed by claim 13 and by a transport and packaging container as claimed by claim 15. Further advantageous embodiments are the subject-matter of the dependent claims.

According to the present invention there is provided a supporting structure for supporting a plurality of containers for substances for medical, pharmaceutical or cosmetic applications, particularly for supporting medication vials or cartridges of relatively large diameters, comprising an upper side and a bottom side opposite to the upper side, a plurality of cup-shaped receptacles for accommodating the containers, and a plurality of resilient positioning devices for positioning and centering the containers in the receptacles, wherein the receptacles extend in a direction perpendicular to the upper side, and supporting protrusions are provided at the bottom ends of the receptacles for retaining the containers in their axial direction when accommodated in the receptacles, wherein the positioning devices are formed as resilient webs protruding from inner side walls of the receptacles radially inwards into the receptacles, each of the receptacles comprising at least two resilient webs disposed at equiangular distances to each other and distributed along the inner side wall of the receptacles, for centering the containers in the receptacles.

Because of the functional separation for retaining the medication containers in axial direction and positioning and centering the medication containers in radial direction, the resilient webs can be formed as relatively weak, flexible ribs that do not need to carry the weight of the medication containers, whereas the retaining protrusions, which have to bear the weight of the medication containers, are preferably formed as stable radial protrusions. For this purpose, the resilient webs may have free ends suspended at and protruding from the side walls of the receptacles. As an alternative, the resilient webs may be formed as bow-like resilient protrusions, having two ends connected with the side walls of the receptacles and a central portion protruding radially inward into the receptacles.

The distributed arrangement of the resilient webs at equiangular distances to each other reliably ensures multi-point bearing of the medication containers in the receptacles, particularly a three-point bearing of the medication containers, and thus a reliably positioning and centering, irrespective of the actual diameter or tolerances of the medication container accommodated. If three or more resilient webs are provided, the front surfaces of the resilient webs may be in linear abutment with the outer surface of the medication containers. On the other hand, if only two resilient webs are provided, the front surfaces of the resilient webs may be arcuate corresponding to the outer diameter of the medication containers to be accommodated.

According to a further embodiment, the resilient webs are formed integrally with the inner side walls of the receptacles, which enables a cost-efficient production of the supporting structure, particularly by plastic injection molding techniques. Here each resilient web preferably has a free end and an opposite coupling end, each free end being directed toward the bottom end of a respective receptacle so that the medication containers may be lowered from above into the receptacles without the risk of getting caught by the resilient webs, and each coupling end being adjoined with a tubular portion of the inner side wall of the respective receptacle. The resiliency of the resilient webs may be performed actually more or less by pivoting the bottom end radially inward and outward about the coupling end, which enables a reliable positioning of a variety of medication containers having significantly different outer diameters.

According to a further embodiment, the free ends of the resilient webs extend substantially in parallel with center lines of the receptacles, which enables a snuggle abutment of the free ends to the bottom portions of the medication containers at low friction, resulting in less material abrasion at the interfaces between resilient webs and medication containers. Here, the coupling ends are preferably slanted with respect to the center lines of the receptacles (or with respect to the side walls) under an acute angle to form slanted insertion surfaces, which help to guide the medication containers upon insertion into the receptacles towards a centered positioning in the receptacles, when the bottom ends of the containers slide along these slanted insertion surfaces. Together with upper tubular portions of the receptacles, these slanted insertion surfaces for funnel-shaped structures, which help to efficiently catch the medication containers wherein lowering the medication containers into the receptacles starts.

According to a further embodiment, the acute angle is within a range of between 2° and 6°, preferably within a range of between 2° and 4° degrees, which helps to catch the medication containers more efficiently upon insertion into the receptacles, and which, on the other hand, enables a proper resiliency of the resilient webs.

According to a further embodiment, the resilient webs are freely suspended within slots formed in the side walls of the receptacles. Here, the resiliency of the resilient is more or less the result of a pivoting of the resilient webs about the above coupling ends. The slots preferably extend in axial direction along the side walls of the receptacles. As will be set-forth in the following in more detail, the slot enables a stacking of several supporting structures, one above the other. In the stacked configuration, the resilient webs of a lower supporting structure extend through the slots of an upper supporting structure and abut the rear sides of resilient webs of an upper supporting structure. Thus, a space-saving storage of a plurality of supporting structures in a stacked configuration is possible. Here, the resilient webs are preferably disposed symmetrically with regard to their associated slots. Preferably, the slots extend from the tubular portion of the receptacles until the bottom ends of the receptacles and are opened toward the bottom side of the supporting structure, so that the receptacles of an upper supporting structure may protrude even deeper into the receptacles of a lower supporting structure, until the upper ends of the resilient webs of a lower supporting structure get in contact with the rear sides of the resilient webs of an upper supporting structure. However, the resilient webs and the associated slots may also be disposed at any other suitable position along the side walls of the receptacles.

According to a further embodiment, the tubular portion of a side wall of a receptacle may extend from the upper side of the supporting structure until at least about 20% of the total axial length of the receptacles, and this length preferably is in a range between approx. 20% and 40% of the total axial length of the receptacles, to provide a sufficient strength and mechanical stability for the receptacles and enable a proper resiliency of the resilient webs. Meanwhile, an inner diameter of the tubular portion is larger than the maximum outer diameter of the containers to be accommodated in the receptacles, so that the medication containers can be efficiently captured upon insertion into the receptacles vertically from above.

According to a further embodiment, the supporting protrusions are formed as annular rings, a hole being formed in the center of the annular supporting protrusions, wherein the resilient webs are freely suspended within slots formed in the side walls of the receptacles and the slots extend until the bottom ends of the receptacles and are opened toward the bottom side of the supporting structure. This enables an even higher packing density in the above stacked configuration, because the receptacles of an upper supporting structure may protrude even deeper into the receptacles of a lower supporting structure, until the upper ends of the resilient webs of a lower supporting structure get in contact with the rear sides of the resilient webs of an upper supporting structure.

According to a further embodiment, at least one protrusion, preferably a single hemispherical protrusion, is formed on the surface of each resilient web, so that in actual use a point-like contact between the protrusion and the outer surface of the medication container is accomplished, which results in less material abrasion and generation of particulars upon insertion or removal of the medication containers.

According to a further embodiment, the side walls are formed by circular side wall portions and a straight separating web extending in parallel with an axial direction of the receptacles and separating two directly adjacent receptacles from each other.

According to a further embodiment, a first circle formed by the circular side wall portion of a first receptacle of the plurality of receptacles, if viewed from above, and a second circle formed by the circular side wall portion of a second receptacle of the plurality of receptacles directly adjacent to the first receptacle, if viewed from above, partially overlap, to thereby enable a high packing density of the medication containers, when supported at the supporting structure.

According to a further embodiment, the side walls are slanted with respect to a center line of the receptacles under a second acute angle, which is in a range of between 2° and 6°, preferably within a range of between 2° and 4° degrees, so that the supporting structure can be ejected easily from an injection mold used for injection molding the supporting structure from a plastic material.

According to a further embodiment, the upper side further comprises a circumferential flat outer rim, which is L-shaped, if viewed in profile, in order to further stiffen the upper side of the supporting structure.

According to a further embodiment, the supporting protrusions of all receptacles span a common plane spaced apart and in parallel with the upper side of the supporting structure. This results in a higher stiffness and mechanical stability of the bottom side of the supporting structures. Further, the supporting structure can be simply put on flat surfaces at a vertical orientation of the medication containers.

According to a further aspect of the present invention there is provided a combined supporting structure consisting of the supporting structure as outlined above and a plurality of containers for substances for medical, pharmaceutical or cosmetic applications accommodated in the supporting structure, particularly vials and cartridges of bigger diameter of glass or plastic.

According to a further embodiment, the maximum outer diameters of the containers are smaller than the opening diameter of the receptacles so that outer circumferential walls of the containers are disposed spaced apart to the side walls of the receptacles, wherein the resilient webs are in resilient abutment with the outer side walls of the containers for centering the containers in the receptacles.

According to a further embodiment, the containers are necked containers including a cylindrical body, particularly vials and cartridges of bigger diameter, wherein the resilient webs extend only along a bottom end of the cylindrical body of the containers and upper ends of the containers protrude beyond the upper side of the supporting structure so that they can be gripped easily for removal.

According to a further aspect of the present invention there is provided an assembly of supporting structures consisting of a plurality of supporting structures as outlined above, which are of identical configuration and which are stacked one above the other so that the receptacles of a first one of the plurality of supporting structures are inserted at least partially into the receptacles of a second one of the plurality of supporting structures, the first and second one of the plurality of supporting structures being disposed stacked directly one above the other.

According to a further embodiment, the receptacles of the first one of the plurality of supporting structures rest directly on the resilient webs of the second one of the plurality of supporting structures supporting structure.

According to a further aspect of the present invention there is provided a transport and packaging container for a plurality of containers for substances for medical, pharmaceutical or cosmetic applications, wherein the transport and packaging container is box-shaped and accommodates the combined supporting structure as outlined above.

According to a further embodiment, an upper side of the transport and packaging container is sterile sealed by a gas-permeable foil, which enables a sterilization of the inner space of the transport and packaging container and of the medication containers accommodated therein by a flow of a sterilizing gas, such as ETO or steam, through the gas-permeable foil.

### Overview on Drawings

The invention will now be described by way of example and with reference to the accompanying drawings, from which further features, advantages and problems to be solved will be-come apparent. In the drawings:
- Figs. 1a to 1d: show a supporting structure for vials and cartridges according to the present invention in a perspective top view, bottom view, perspective bottom view and top view;
- Fig. 1e: is a cross section of a receptacle of the supporting structure for vials and cartridges of Fig. 1a;
- Figs. 2a to 2c: show a supporting structure according to the present invention, which supports a plurality of vials, in an exploded perspective top view, in a perspective top view and in a top view;
- Fig. 2d: is a cross section of a receptacle of the supporting structure of Fig. 1a along A-A, which accommodates a vial of a relatively large outer diameter;
- Fig. 2e: is a cross section of a receptacle of the supporting structure of Fig. 1a along A-A, which accommodates a vial of a smaller outer diameter;
- Fig. 2f: shows in an exploded perspective view a packaging unit consisting of a transport and packing container, a supporting structure according to the present invention, which accommodates a plurality of vials, and of a gas-permeable sheet sealing the upper end of the transport and packing container;
- Fig. 2g: shows the packaging unit of Fig. 2f in an assembled state;
- Fig. 3a: shows in a perspective top view a stack of supporting structures according to the present invention, stacked one in another;
- Fig. 3b: is a cross-sectional view of the stack of supporting structures of Fig. 3a; and
- Fig. 3c: is a perspective partial sectional view of the stack of supporting structures of Fig. 3a.

In the drawings, the same reference numerals designate identical or substantially equivalent elements or groups of elements.

### Detailed description of preferred embodiments

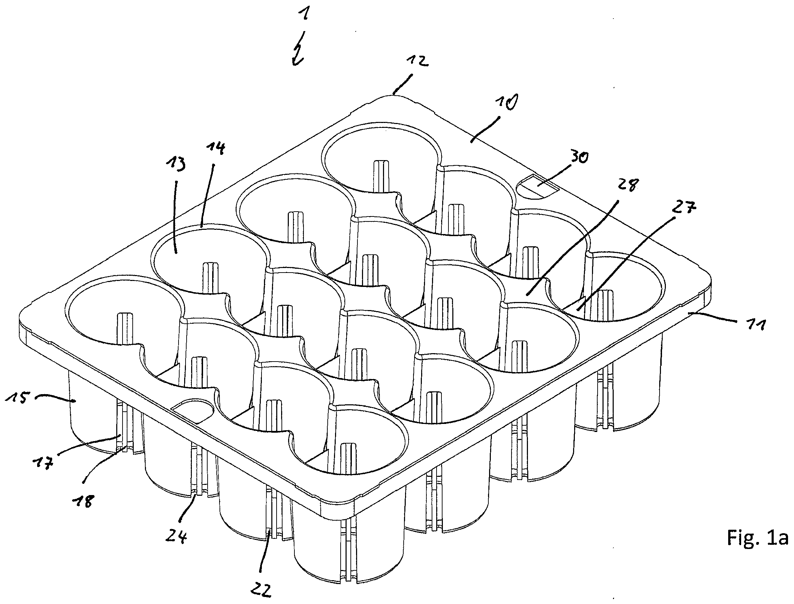

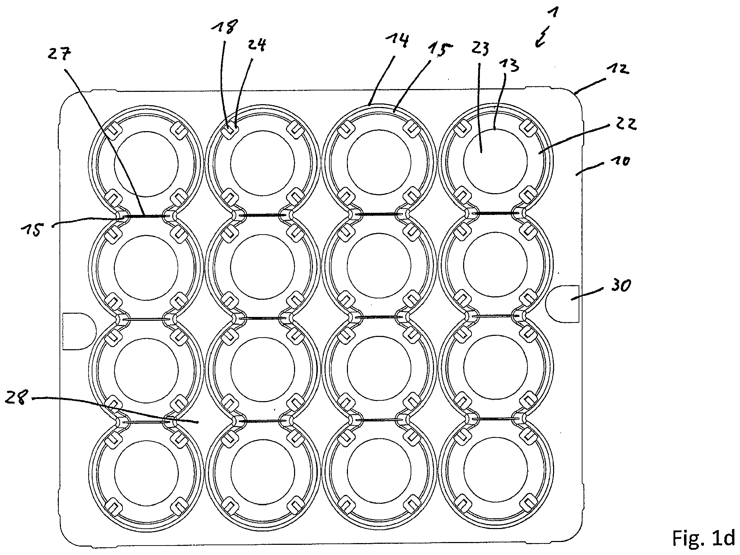

As shown in Fig. 1a, the supporting structure 1 is formed as a nest for nested packaging solutions, for accommodating a plurality of vials (not shown) in cup-shaped receptacles 13. The supporting structure 1 has a generally flat upper side 10, the edge of which surrounds the plurality of receptacles 13. The receptacles 13 are arranged in a regular arrangement, as shown in Fig. 1a, along rows and columns extending perpendicular thereto, which eases a precise positioning of processing devices used for processing the vials relative to the vials, e.g. of a matrix of filling needles for filling the vials while being accommodated in the receptacles 13.

The cup-shaped receptacles 13 are formed by side walls 15 that are circular, if viewed in a top view. Along a first direction of the supporting structure 1 the receptacles 13 are separated by plate-shaped separation walls 27 separating directly adjacent receptacles. Along a second direction, which is perpendicular to this first direction, the receptacles 13 are spaced apart from each other so that rhomb-shaped connecting portions 28, which are flush with the upper side 10, are formed between columns of receptacles 13.

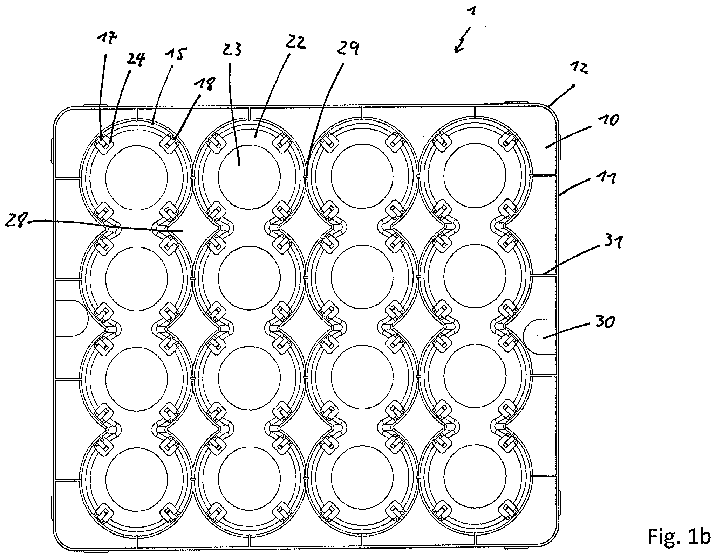

As shown in the top view of Fig. 1b, each side wall 15 of a receptacle 13 basically forms a circle, if viewed in top view. Here, a first circle formed by the circular side wall 15 of a first receptacle 13 of the plurality of receptacles, if viewed from above, and a second circle formed by the circular side wall of a second receptacle 13 of the plurality of receptacles directly adjacent to this first receptacle 13, if viewed from above, partially overlap in the direction of the columns of receptacles. Thus, the base area of the supporting structure 1 is minimized and a high packing density can be accomplished. The afore-mentioned plate-shaped separation walls 27 are used for preventing a direct contact of vials accommodated in directly adjacent receptacles.

Preferably, the entire supporting structure 1 shown in Fig. 1a is formed integrally from a plastic material by injection molding, including the separation walls 27. The side walls 15 forming the receptacles 13 are slanted radially inwards under a small acute angle relative to a middle axis of the receptacles 13 from the top side 10 to their bottom ends, e.g. by an angle of 2 to 6 degrees, more preferably by an angle of 2 to 4 degrees, to ease an ejection of the supporting structure 1 out of a mold used for injection-molding of the supporting structure 1.

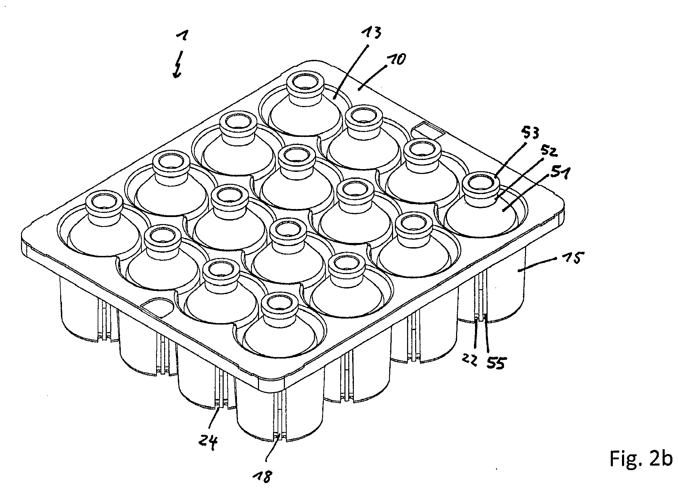

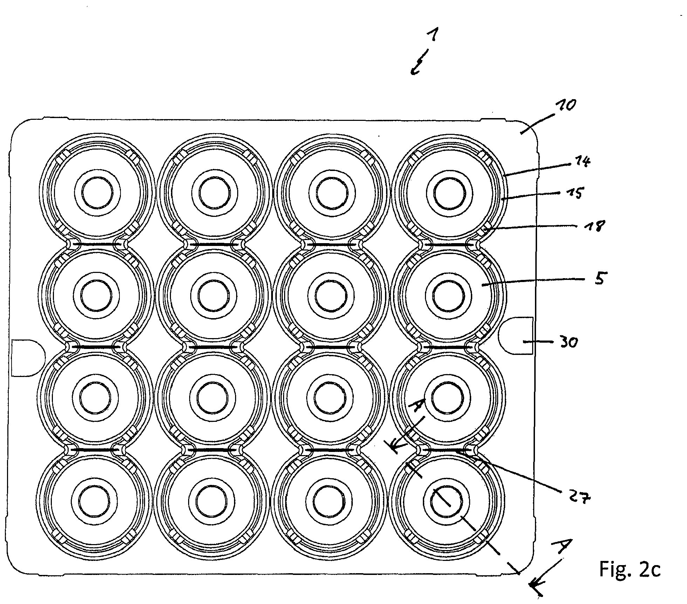

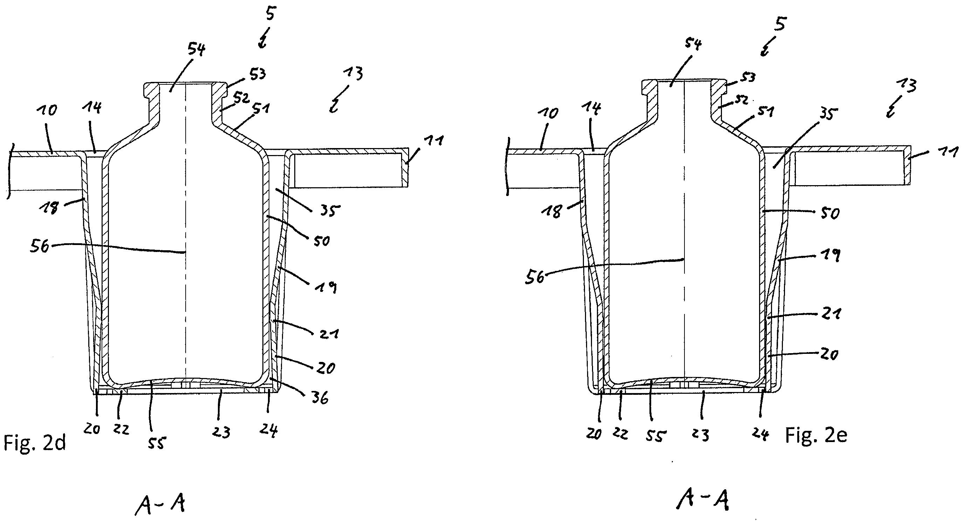

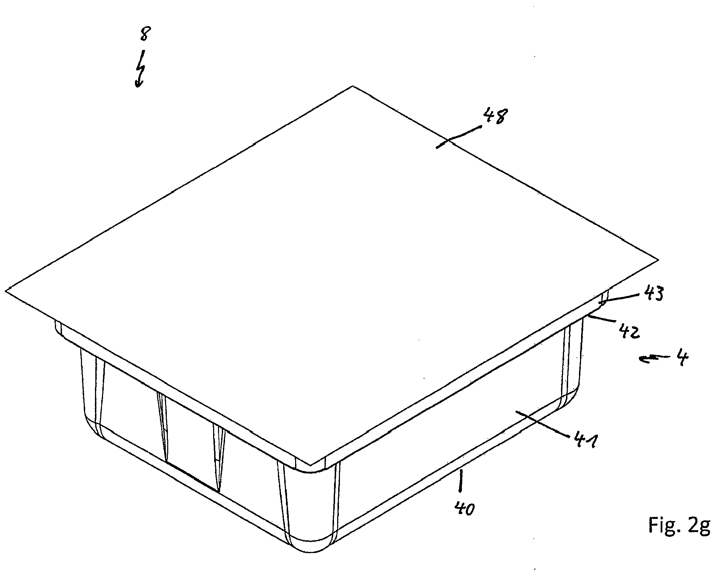

As shown in Fig. 1a, axial slots 17 extending in parallel with the central axis of the receptacles are formed in the side walls 15. In the embodiment shown in Fig. 1a, these axial slots 17 extend up to annular retaining protrusions 22 formed at the bottom ends of the receptacles 13. As shown in the top view of Fig. 1b, the retaining protrusions 22 extend radially inward from the side walls 15, for retaining vials accommodated in the receptacles 13 in axial direction by supporting the bottoms of the vials (see Figs. 2d and 2e).

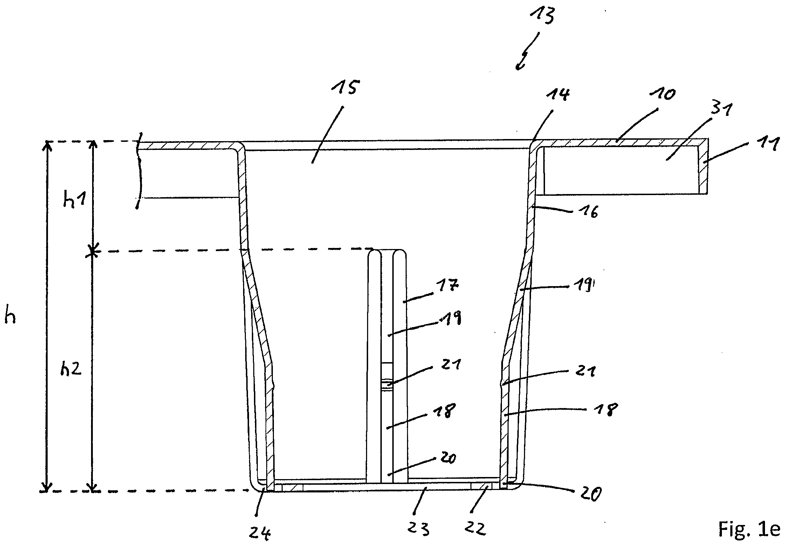

As shown in Figs. 1a and 1b, the axial slots 17 are disposed at equiangular distances along the side walls 15, and are aligned with rectangular recesses formed in the annular retaining protrusions 22. In each axial slot 17 a resilient web 18 is disposed at a central position, which extends in axial direction. Each resilient web 18 has a free end (bottom end) 20 that is freely suspended in the axial slot 17 and is directed to the bottom end of a respective receptacle 13, and an opposite coupling end that is adjoined with a tubular portion 16 of the inner side wall 15 of the respective receptacle 13. As shown in Fig. 1e, the axial length h1 of the tubular portion 16, if measured in projection on the middle axis of the receptacle, is less than the axial length h2 of the resilient web 18, which ensures a certain degree of resiliency.

The resilient webs 18 are used as positioning devices for positioning and centering vials accommodated in the receptacles 13. As the resilient webs 18 are disposed in a point-symmetric configuration and at equiangular distances along the side walls 15, a stable, centered three-point or multi-point bearing of the vials in the receptacles can be accomplished.

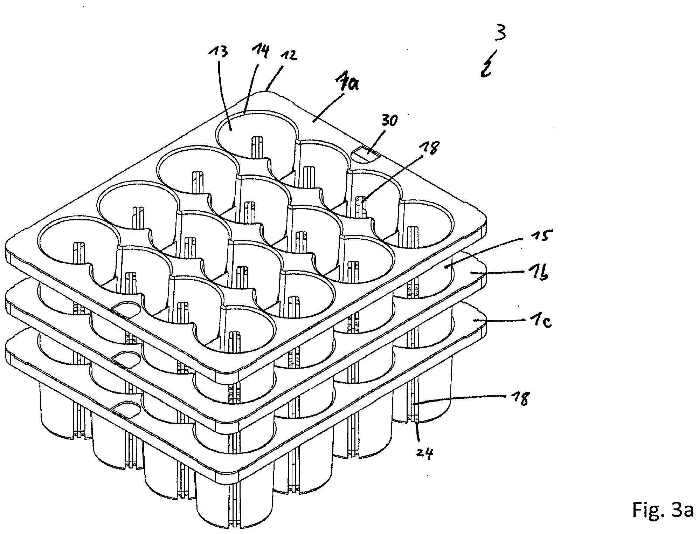

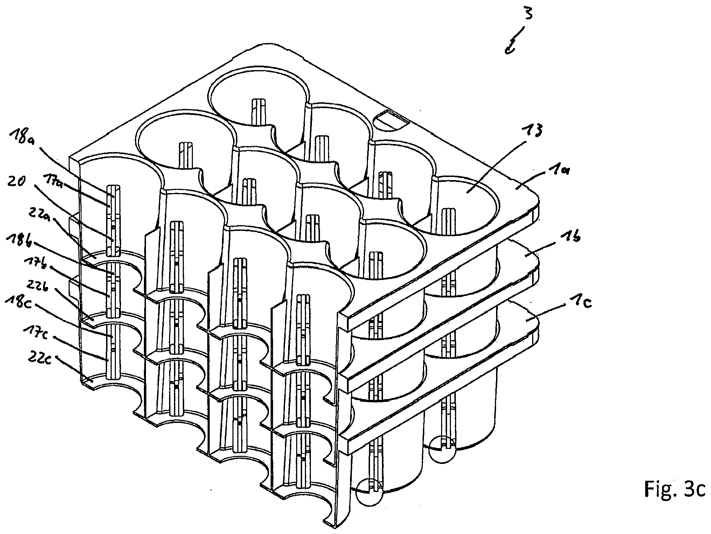

As the axial slots 17 are aligned with the rectangular recesses formed in the annular retaining protrusions 22, also each of the resilient webs 18 is aligned with a corresponding one of these rectangular recesses formed in the annular retaining protrusions 22, which makes it possible to stack the supporting structures one above the other, as described below in more detail with reference to Figs. 3a-3c.

As will become apparent to the person skilled in the art, the resilient webs 18 do not necessarily need to extend up to the retaining protrusions 22, but may be provided at a proper axial position of the receptacles 13 for positioning and centering the vials 5 at a suitable position. E.g. for a 2ml vial the resilient webs 18, particularly the protrusions 21, may contact the cylindrical body of the vial near a center point of the cylindrical body as well. The resilient webs 18 thus may extend only over a relatively short distance along the side walls 15 and particularly do not need to extend up to the bottom ends of the receptacles 13 (i.e. the retaining protrusions 22). It is noted that, if the axial slots 17 do not extend up to the retaining protrusions 22, there will be no need for the presence of the radial slots 24 in the retaining protrusions. Particularly, the radial slots 24 need not be aligned with the axial slots 17 in the side walls 15 of the receptacles 13. Rather, for enabling a stacking of the stacking of the supporting structures 1 it is sufficient that the axial lengths of the resilient webs 18 is not longer than the axial lengths of the axial slots 17.

Further details of the configuration of the resilient webs 18 and receptacles 13 are shown in Fig. 1e. The upper ends of the resilient webs 18 are slanted at an acute angle of e.g. 15 to 26 degrees relative to the vertical side walls 15 and form slanted insertion surfaces 19, as outlined below in more detail with reference to Figs. 2d and 2e. The slanted insertion surfaces 19 are followed by a vertical portion that extends up to the bottom end 20 of the resilient webs 18. The bottom ends 20 of the resilient webs 18 are freely suspended in the radial slots 24 formed in the retaining protrusions 22 and neither contact the side wall 15 nor the retaining protrusion 22 in an unloaded position. Near the transition region between the slanted insertion surface 19 and the vertical portion of the resilient web 15, a hemispherical protrusion 21 is formed, which forms in most practical cases the actual contact point between the vial to be positioned and the resilient web 18.

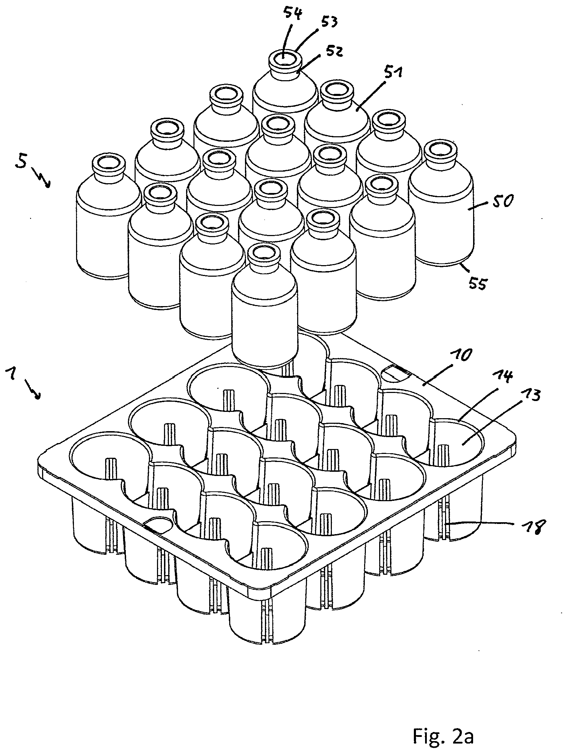

The general configuration of a vial to be positioned is shown in Fig. 2a. The vials 5 consist of a generally cylindrical body 50 of a constant outer diameter, which is followed by a slanted shoulder portion 51, a vertical constricted neck portion 52, which is followed by a widened upper rim 53 that forms the upper end of the vial and encloses a filling opening 54. The bottom end of the vial 5 is closed by a bottom 55. A vial as shown in Fig. 2a merely serves as an example for a medication container to be accommodated by the receptacles 13. It will become apparent to the person skilled in the art, that other types of medication containers of generally cylindrical shape may be accommodated as well by the receptacles 13 of the supporting structure, particularly cartridges, which may have relatively large diameters.

If accommodated in a receptacle 13, as shown in Fig. 2d, the bottom 55 is directly supported on the annular retaining protrusion 22, whereas the hemispherical protrusions 21 and/or the bottom ends 20 of the resilient webs 18 abut the lower part of the cylindrical body 50, depending on the actual outer diameter of the vial 5. Whereas Fig. 2d shows the accommodation of a vial 5 of a relatively large outer diameter, Fig. 2e shows the accommodation of a vial 5 of a relatively small outer diameter. In the case of Fig. 2d, the bottom ends 20 of the resilient webs 18 cannot project from the side wall 15 inwards over a large distance so that an actual point contact is established between the hemispherical protrusions 21 and the lower part of the cylindrical body 50. The bottom ends 20 of the resilient webs 18 extend radially outward again, so that a narrow gap 36 may be formed between the bottom ends of the resilient webs 18 and the lower part of the cylindrical body 50. In the case of Fig. 2e, where a vial of a smaller outer diameter is accommodated in the receptacle, the bottom ends 20 of the resilient webs 18 protrude a little further into the receptacle 13 so that the bottom ends 20 of the resilient webs 18 may abut the lower part of the cylindrical body 50 over a certain distance.

As shown in Fig. 2d, if a vial 5 of a largest possible diameter is accommodated in a receptacle, the insertion surfaces 19 formed at the upper ends of the resilient webs 18 are slanted at an acute angle of e.g. 15 degrees relative to the vertical side walls 15. On the other hand, as shown in Fig. 2e, if a vial 5 of a smallest possible diameter with resilient abutment of a resilient web 18 is accommodated in a receptacle, the insertion surfaces 19 formed at the upper ends of the resilient webs 18 are slanted at an acute angle of e.g. 26 degrees relative to the vertical side walls 15.

If a vial 5 is lowered vertically from above into a receptacle 13, first the lower part of the cylindrical body 50 is captured by a funnel-shaped structure formed by the rounded edge 14 at the upper end of the receptacle 13, by the tubular portion 16 of the side wall 15 and by the slanted insertion surface 19. The maximum diameter of this funnel-shaped structure is larger than the largest outer diameter of the vials to be accommodated, so that vials are reliably caught by the funnel-shaped structure. Upon further lowering the vial 5, the lower part of the cylindrical body 50 is further guided by the slanted insertion surfaces 19 into the receptacle to a centered position. Finally, the lower part of the cylindrical body 50 gets in contact with the bottom ends of the resilient webs 18 and spreads apart the resilient webs 18. Because the resilient webs 18 are disposed at equiangular distances, the vial 5 remains centered. When the vial 5 is further lowered, the lower part of the cylindrical body 50 slides along the bottom ends 20 of the resilient webs 18 until the bottom 55 rests on the annular retaining protrusion 22. Due to the resiliency of the webs 18 a variety of vials 5 having significantly different outer diameters can be reliably positioned and centered in the receptacles 13. The resiliency of the webs 18 efficiently damps vibrations and a rattling of vials 5 accommodated in the receptacles 13. When the vials 5 are fully accommodated in the receptacles 13, at least the widened upper rims 53 of the vials 5 project beyond the upper side 10 of the supporting structure 1 so that the vials 5 can be gripped easily for removal out of the receptacles 13.

Actually, the main portion of the resiliency of the resilient webs 18 is defined by the material characteristics and configuration of the slanted insertion surfaces 19 suspending the bottom ends 20 of the resilient webs 18, and can be easily adjusted as desired. When the vials 5 are inserted into the receptacles 13 from above, actually the upper insertion surfaces 19 are flexed resiliently, whereas the bottom ends 20 of the resilient webs 18 always extend more or less in the axial direction and are not significantly flexed relative to the slanted insertion surfaces 19 in actual use. In other words, the resilient webs 18 are more or less pivoted resiliently about the region, where the slanted insertion surfaces 19 adjoin the tubular portions 16 of the receptacles 13. To offer a proper resiliency, in most applications the axial length h2 of the resilient webs 18 will be much larger than the axial length h1 of the tubular portion 16.

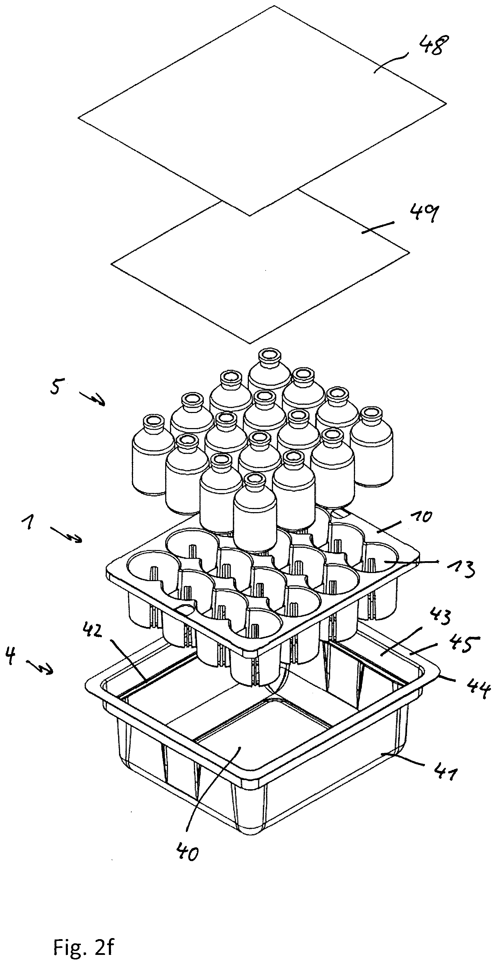

As shown in Fig. 2f, a supporting structure 1 (nest) accommodating a plurality of vials 5 in a precisely defined geometric configuration can be accommodated in a box-shaped transport and packaging container 4 (tub), which is sterile sealed by a sterile sheet 48 bonded onto the upper flange 45 of the transport and packaging container 4. The sheet 48 may be gas-permeable, in particular a web of synthetic fibers such as high density poly ethylene (HDPE) or a Tyvek® protective film, which enables a sterilization of the vials 5 accommodated and packaged in the transport and packaging container 4 through the film 48. A Tyvek^{®} sheet 49 may be disposed between sheet 48 and the vials 5, to prevent intrusion of particles into the vials 5 even after removal of sheet 48 from the transport and packaging container 4, e.g. before the further processing of the vials 5 actually starts.

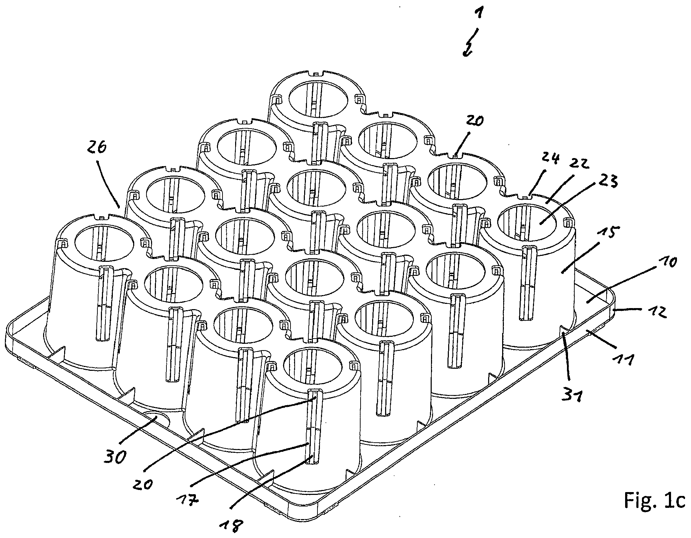

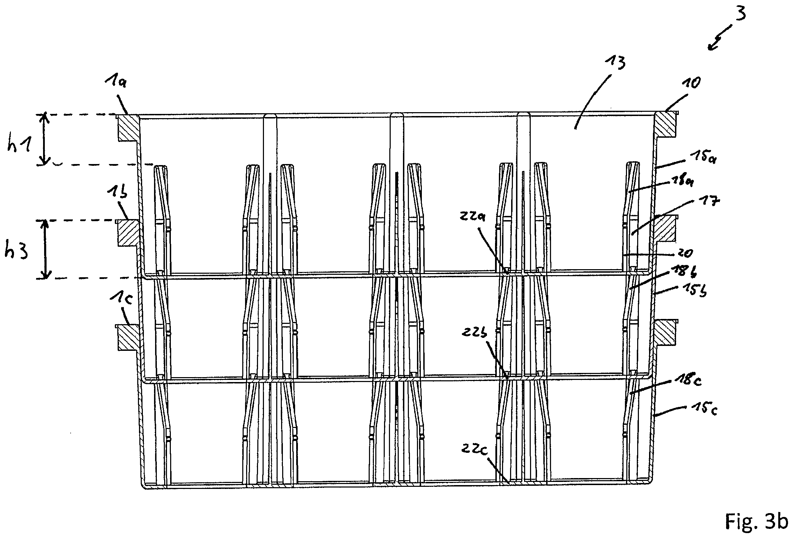

As shown in the top view of Fig. 1b, the axial slots 17 and resilient webs 18 of all receptacles 13 are disposed at exactly the same positions along the side walls 15. Because interspaces 26 (see Fig. 1c) are provided between neighboring cup-shaped receptacles 13, particularly between neighboring columns of cup-shaped receptacles 13, because the side-walls 15 of the receptacles 13 are slanted radially inward and because of the above configuration of the axial slots 17 and resilient webs 18, a plurality of supporting structures 1 as outlined above can be stacked one above the other to form a stack 3 of nests or supporting structures 1 as shown in Figs. 3a-3c. More specifically, Fig. 3a shows the stacking of three supporting structures designated by reference numerals 1a, 1b, 1c from top to bottom. As shown in Fig. 3b, in this stacked configuration the side walls 15a of the uppermost nest 1a are partially accommodated in the receptacles of the middle nest 1b, whereas the side walls 15b of the middle nest 1b are partially accommodated in the receptacles of the bottommost nest 1c. In this stacked configuration, the retaining protrusions 22a of the uppermost nest 1a rest directly on the upper ends of the resilient webs 18b of the middle nest 1b, particularly on the upper ends of the slanted insertion surfaces of the resilient webs 18b. This also holds for the retaining protrusions 22b of the middle nest 1b, which rest directly on the upper ends of the resilient webs 18c of the bottommost nest 1c, particularly on the upper ends of the slanted insertion surfaces of the resilient webs 18c.

In this stacked configuration, the upper ends of the resilient webs 18b of the middle nest 1b, particularly the upper ends of the slanted insertion surfaces of the resilient webs 18b, directly contact the rear sides of the bottom ends 20 of the resilient webs 18a of the upper nest 1a, which is possible, because the axial slots 17 of the upper nest 1a extend until the bottom end of the receptacles 13 of the upper nest and because the radial slots 24 are provided in the retaining protrusions 22 of the upper nest (see Fig. 1b). Here, it may be of advantage, if the bottom ends 20 of the resilient webs 18 extend into these radial slots 24, because the radially inward flexing of the resilient webs 18 is then delimited by these radial slots. Thus, in the stacked configuration of Fig. 3b the upper ends of the resilient webs 18b of the middle nest 1b may flex the resilient webs 18a of the upper nest 1a radially inward, but this radially inward flexing may be delimited by the radial slots 24, to thereby fix the positional relationship between two adjacent nests in this stacked configuration. This positional relationship is, of course, also defined by the direct abutment of the receptacle side walls 15 of two adjacent nests in the stacked configuration.

In this manner, many supporting structures 1 may be stacked one above the other, which offers a space-saving solution for temporary storage of many supporting structures 1 with high packing density, e.g. after or during processing of the medication containers in a clean room. The distance h3 over which the receptacles of an upper supporting structure extend into the receptacles of a lower supporting structure basically corresponds to the axial length h1 of the tubular portions 16 of the side walls 15. Thus, for obtaining a high packing density in the stacked configuration, the axial length h1 of the tubular portions 16 should be maximized, which also ensure a proper mechanical stiffness of the receptacles 13. On the other hand, for offering the possibility to accommodate a variety of different vials having significantly different outer diameters and for offering a proper resiliency of the resilient webs 18, the lengths of the slanted insertion surfaces should project over a certain minimum distance radially inward. Thus, the actual design of the receptacles 13, axial slots 17 and resilient webs 18 is a trade-off between the packing density of the stacked assembly of supporting structures to be achieved and the actual variation to be expected of the outer diameters of the vials to be accommodated.

As shown in Fig. 1c, the supporting structure 1 is further stiffened by the following measures on the back side of the upper side 10:
- the edge of the upper side 10 is followed by circumferential side wall 11, which projects perpendicular from the upper side 10;
- the outer cup-shaped receptacles 13 are connected with the side wall 11 via stiffening ribs 31;
- the annular protrusions 22 are connected with each other column-wise, leaving an interspace 26 along columns of cup-shaped receptacles 13, which is required along at least one direction of the regular arrangement of receptacles for enabling the stacking of supporting structures;
- as can be seen in the top view of Fig. 1b, in the row-wise direction adjacent receptacles 13 contact each other at contact portions 29, which may be formed as very narrow connecting webs, connecting the rhomb-shaped connecting portions 28 with each other.

As shown in Fig. 1c, central holes 23 are formed in the center of the annular retaining protrusions 22 so that the vials can be pushed upward by means of rods or the like acting onto the bottoms of the vials.

As will become apparent to the person skilled in the art a further aspect of the present invention, which may also be claimed explicitly by means of an independent patent claim as a separate independent invention, relates to a computer-readable or processor-readable data file, also for transmission over networks, such as internal computer networks or over the Internet, comprising instructions or control commands which, when loaded by a computer or a processor, cause a 3D-printer, under control of the computer or processor, to print out a supporting structure as disclosed in the present application in three-dimensional shape using a suitable material, in particular, a plastic material.

### List of reference numerals

- 1: supporting structure / nest
- 1a, 1b, 1c: supporting structure / nest

- 3: stack of nests or supporting structures
- 4: tub / transport and packaging container
- 5: vial / necked container

- 8: packaging unit

- 10: upper side
- 11: side wall of supporting structure / nest 1
- 12: rounded corner
- 13: receptacle
- 14: rounded edge at upper end of receptacle 13
- 15: side wall of receptacle 13
- 15a, 15b, 15c: side wall of receptacle
- 16: tubular portion of side wall 15
- 17: axial slot
- 17a, 17b, 17c: axial slot
- 18: resilient web
- 18a, 18b, 18c: resilient web
- 19: slanted insertion surface
- 20: bottom end of resilient web 18
- 21: protrusion
- 22: retaining protrusion
- 22a, 22b, 22c: retaining protrusion
- 23: central hole
- 24: radial slot

- 26: interspace between neighboring receptacles 13
- 27: separation wall
- 28: connecting portion
- 29: contact portion
- 30: access opening
- 31: stiffening web

- 35: gap
- 36: gap

- 40: bottom
- 41: bottom side wall
- 42: step
- 43: upper side wall
- 44: rounded corner
- 45: upper flange

- 48: gas-permeable sheet
- 49: gas-permeable sheet

- 50: cylindrical body
- 51: shoulder portion
- 52: constricted neck portion
- 53: widened upper rim
- 54: filling opening
- 55: bottom
- 56: centre line

- h: total height of receptacle 13
- h1: height of tubular portion 16
- h2: height of resilient web 18
- h3: dipping depth

## Claims

1. A supporting structure for supporting a plurality of containers (5) for substances for medical, pharmaceutical or cosmetic applications, comprising
an upper side (10) and a bottom side opposite to the upper side,
a plurality of cup-shaped receptacles (13) for accommodating the containers, and
a plurality of resilient positioning devices (18) for positioning and centering the containers in the receptacles, wherein
the receptacles (13) extend in a direction perpendicular to the upper side (10), and supporting protrusions (12) are provided at the bottom ends of the receptacles for retaining the containers in their axial direction when accommodated in the receptacles, wherein
the positioning devices are formed as resilient webs (18) protruding from inner side walls of the receptacles (13) radially inwards into the receptacles,
each of the receptacles comprising at least two resilient webs (18) disposed at equiangular distances to each other and distributed along the inner side wall (15) of the receptacles, for centering the containers in the receptacles, wherein
the resilient webs (18) are formed integrally with the inner side walls (15) of the receptacles, each resilient web having a free end (20) and an opposite coupling end (19), each free end being directed toward the bottom end of a respective receptacle and each coupling end being adjoined with a tubular portion (16) of the inner side wall (15) of the respective receptacle.

2. The supporting structure as claimed in claim 1, wherein the free ends (20) of the resilient webs extend substantially in parallel with center lines of the receptacles and wherein the coupling ends are slanted with respect to the center lines under an acute angle to form slanted insertion surfaces (19) for guiding the containers (5) upon insertion into the receptacles (13) towards a centered positioning in the receptacles, wherein
the acute angle preferably is within a range of between 15° and 26°, more preferably within a range of between 15° and 23° degrees.

3. The supporting structure as claimed in any of the preceding claims, wherein the resilient webs (18) are freely suspended within slots (17) formed in the side walls (15) of the receptacles, wherein the resilient webs are disposed symmetrically with regard to their associated slots.

4. The supporting structure as claimed in claim 3, wherein the slots extend from the tubular portion of the receptacles until the bottom ends of the receptacles and are opened (24) toward the bottom side of the supporting structure.

5. The supporting structure as claimed in claim 3 or 4, wherein the tubular portion extends from the upper side of the supporting structure until at least 20% of the total axial length of the receptacles, this distance being more preferably in a range between 20% and 40% of the total axial length of the receptacles, wherein an inner diameter of the tubular portion is larger than the maximum outer diameter of the containers to be accommodated in the receptacles.

6. The supporting structure as claimed in any of the preceding claims, wherein the supporting protrusions (22) are annular, a hole (23) being formed in the center of the annular supporting protrusions, wherein the resilient webs (18) are freely suspended within slots (17) formed in the side walls (15) of the receptacles and the slots extend until the bottom ends of the receptacles and are opened toward the bottom side of the supporting structure.

7. The supporting structure as claimed in any of the preceding claims, wherein at least one protrusion (21), preferably a single hemispherical protrusion, is formed on the surface of each resilient web (18).

8. The supporting structure as claimed in any of the preceding claims, wherein the side walls (15) are formed by circular side wall portions and a straight separating web (27) extending in parallel with an axial direction of the receptacles and separating two directly adjacent receptacles (13) from each other, wherein
a first circle formed by the circular side wall portion of a first receptacle of the plurality of receptacles, if viewed from above, and
a second circle formed by the circular side wall portion of a second receptacle of the plurality of receptacles directly adjacent to the first receptacle, if viewed from above, partially overlap.

9. The supporting structure as claimed in claim 8 or 9, wherein the side walls (15) are slanted with respect to a center line of the receptacles under a second acute angle, which is in a range of between 2° and 6°, preferably within a range of between 2° and 4° degrees.

10. The supporting structure as claimed in any of the preceding claims, wherein
the upper side (10) further comprises a circumferential flat outer rim, which is L-shaped (11), if viewed in profile, and/or
wherein the supporting protrusions (22) of all receptacles span a common plane spaced apart and in parallel with the upper side of the supporting structure.

11. A combined supporting structure consisting of the supporting structure as claimed in any of the preceding claims and a plurality of containers (5) for substances for medical, pharmaceutical or cosmetic applications accommodated in the supporting structure, wherein
the maximum outer diameters of the containers (5) are smaller than the opening widths of the receptacles (13) so that outer circumferential walls (50) of the containers are disposed spaced apart to the side walls (15) of the receptacles, wherein the resilient webs (18) are in resilient abutment with the outer side walls (50) of the containers for centering the containers in the receptacles.

12. The combined supporting structure as claimed in claim 11, wherein the containers are necked containers including a cylindrical body, wherein the resilient webs extend only along a bottom end of the cylindrical body of the containers and upper ends of the containers protrude beyond the upper side of the supporting structure.

13. An assembly (3) of supporting structures (1) consisting of a plurality of supporting structures as claimed in any of claims 1 to 10, which are of identical configuration and which are stacked one above the other so that the receptacles of a first one (1a) of the plurality of supporting structures are inserted at least partially into the receptacles of a second one (1b) of the plurality of supporting structures, the first and second one of the plurality of supporting structures being disposed stacked directly one above the other.

14. The assembly of supporting structures as claimed in claim 13, wherein the receptacles (13) of the first one (1a) of the plurality of supporting structures rest directly on the resilient webs (18b) of the second one (1b) of the plurality of supporting structures

15. A transport and packaging container (4) for a plurality of containers for substances for medical, pharmaceutical or cosmetic applications, wherein the transport and packaging container is box-shaped and accommodates the combined supporting structure as claimed in claim 11 or 12, wherein an upper side of the transport and packaging container is prerably sterile sealed by a gas-permeable foil (48).
